# EUROPEAN PATENT APPLICATION

(11) **EP 3 162 400 A1**
(43) Date of publication of application: **03.05.2017**
(21) Application number: 15306744.2
(22) Date of filing: 02.11.2015
(51) Int. Cl.: A61M 5/32, A61M 5/158

(54) **NEEDLE ASSEMBLY**

(71) Applicant: SANOFI, 75008 Paris (FR)
(72) Inventor: The designation of the inventor has not yet been filed
(74) Representative: Schupp, Bernhard

(57) **Abstract**

The invention relates to a needle assembly (1, 101, 201, 301, 401, 501, 601), comprising a hollow needle (3, 103, 203, 303, 403, 503, 603) with a
flow channel (3.1, 103.1, 203.1, 303.1, 403.1, 503.1, 603.1) and a needle hub (2, 102, 202, 302, 402, 502, 602).

At least one pressure sensitive element (4, 104, 204, 304, 404, 504, 604) is adapted to detect an occlusion in the flow channel (3.1, 103.1, 203.1, 303.1, 403.1, 503.1, 603.1), wherein the pressure sensitive element (4, 104, 204, 304, 404, 504, 604) is modifiable if a pressure in the flow channel (3.1, 103.1, 203.1, 303.1, 403.1, 503.1, 603.1) exceeds a predetermined value.

## Description

### Technical Field

The invention relates to a needle assembly.

### Background of the Invention

In injection devices, there is a trend toward the use of smaller and smaller gauge needles in the type of needles typically used for self-injection. While a small gauge needle typically reduces the pain associated with the procedure, their use can result in an increased risk of partial or complete occlusion of the needle, especially when used in conjunction with higher concentrated insulin formulations.

One potential cause of needle occlusion is solidification of the drug formulation within the inner bore of the needle. This may occur if the needle is left in-situ following use, or if a user fits a needle in preparation for taking an injection later that day. It is known that particular drug formulations, especially those that are water-based, may be more at risk of solidification, in particular storage situations. One such example might be higher concentration insulin formulations.

Thus, there remains a need for reducing the negative impact of potential occlusions of an injection needle to enable reliability and robustness of medication.

### Summary of the Invention

In view of the aforementioned need, it may be understood as an object of the present invention to provide for an improved needle assembly.

The object is achieved by a needle assembly according to claim 1.

Exemplary embodiments of the invention are given in the dependent claims.

A needle assembly comprises a hollow needle, in particular a hypodermic hollow needle, defining a flow channel and a needle hub. According to the invention, at least one pressure sensitive element is adapted to detect an occlusion in the flow channel, wherein the pressure sensitive element is modifiable if a pressure in the flow channel exceeds a predetermined value. Herein, the pressure sensitive element means an element that changes at least one parameter by variation of a pressure that the pressure sensitive element is subjected to. The needle assembly enables a detection of an occlusion in an injection needle with a pressure operable mechanism that indicates the occlusion to a user and thus minimizes a risk for the user to receive an insufficient dose.

In an exemplary embodiment, the pressure sensitive element comprises a valve that is configured for opening if the pressure in the flow channel exceeds the predetermined value.

The valve may comprise a plug that is positively and/or non-positively fitted within a recess that is arranged in a bypass channel of the needle hub, wherein the bypass channel is fluidly connected to the flow channel, and wherein the plug is pushed out of the recess if the pressure in the flow channel exceeds the predetermined value. The recess couples the bypass channel with an outside of the needle hub. As a consequence, the fluid partially leaks out of the flow channel through the bypass channel towards the outside of the needle hub. For example, the fluid leaks out of the needle hub to a skin of the user who is thus made aware of the occlusion.

Furthermore, the valve may comprise a plug that is coupled to a spring, wherein the plug is positively and/or non-positively fitted within an opening arranged in a lateral surface of the needle, and wherein the plug is pushed out of the opening if the pressure in the flow channel exceeds the predetermined value. For example, the spring is movable perpendicularly with respect to the flow channel, wherein the spring is tensioned when the plug is pushed out of the opening.

In a further exemplary embodiment, the valve is coupled to an inner indicator that is arranged within the needle hub and fluidly connected to the flow channel when the valve is opened. The inner indicator may generate a visual, audible and/or tactile feedback when the valve is opened. To generate a visual feedback, the inner indicator may comprise a chemical indicator such as an acid and/or base sensitive indicator that changes a colour by coming in contact with the fluid leaked out from the flow channel.

In a further exemplary embodiment, the valve is coupled to an inner indicator and a further inner indicator is provided, wherein the inner indicator generates a visual and/or audible and/or tactile feedback when the valve is opened, and wherein the further inner indicator generates a visual and/or audible and/or tactile feedback when a fluid is leaked out of a cartridge of a drug delivery device. This embodiment is suited to differentiate between a clogged or blocked needle and a leaking cartridge.

According to an alternative embodiment, the pressure sensitive element comprises a predetermined breaking point that is arranged in a lateral surface of the needle, wherein the predetermined breaking point breaks if the pressure within the flow channel exceeds the predetermined value.

In a further alternative embodiment, the pressure sensitive element comprises a flexible section of the needle that forms a section of a flow channel with a variable diameter, wherein the diameter of the flexible section increases if the pressure in the flow channel exceeds the predetermined value. Therefore, the flexible section may be made from an elastic material such as rubber.

Additionally, the diameter of the flexible section is detectable by a sensor that is coupled to the flexible section. The sensor may be one of an optical, ultrasonic, magnetic or electrostatic sensor, wherein a visual, audible and/or tactile feedback is issued if the sensor detects a diameter of the flexible section exceeding a predetermined value.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given herein below and the accompanying drawings which are given by way of illustration only, and thus, are not limitative of the present invention, and wherein:
- Figure 1: is a schematic longitudinal section of a needle assembly with a pressure sensitive element in an exemplary embodiment,
- Figure 2: is a schematic longitudinal section of the needle assembly according to figure 1 with a clogged needle,
- Figure 3: is a schematic longitudinal section of a needle assembly with a pressure sensitive element in a further exemplary embodiment,
- Figure 4: is a schematic longitudinal section of the needle assembly according to figure 3 with a clogged needle,
- Figure 5: is a schematic longitudinal section of a needle assembly with a pressure sensitive element in a further exemplary embodiment,
- Figure 6: is a schematic longitudinal section of the needle assembly according to figure 5 with a clogged needle,
- Figure 7: is a schematic longitudinal section of a needle assembly with a pressure sensitive element in an alternative exemplary embodiment,
- Figure 8: is a schematic longitudinal section of the needle assembly according to figure 7 with a clogged needle,
- Figure 9: is a schematic longitudinal section of a needle assembly with a pressure sensitive element in an alternative exemplary embodiment,
- Figure 10: is a schematic longitudinal section of the needle assembly according to figure 9 with a clogged needle,
- Figure 11: is a schematic longitudinal of a needle assembly with a pressure sensitive element in a further alternative exemplary embodiment and
- Figure 12: is a schematic longitudinal section with a needle assembly with a pressure sensitive element in a further alternative exemplary embodiment.

Corresponding parts are marked with the same reference symbols in all figures.

### Detailed Description

In the present application, when the term "distal section/end" is used, this refers to the section/end of the components, which under use is located closest to a drug delivery site of the patient. Correspondingly, when the term "proximal section/end" is used, this refers to the section/end of the components which under use is located the furthest away to the drug delivery site of the patient.

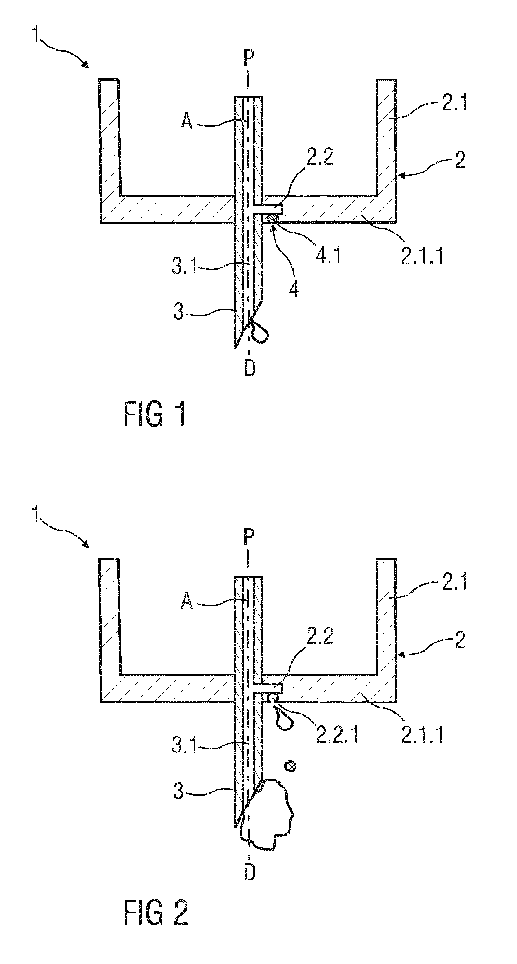

The **figures 1 and 2** show longitudinal sections of a needle assembly 1 in an exemplary embodiment.

The needle assembly 1 comprises a needle hub 2, a hypodermic double ended hollow needle 3 held in the needle hub 2 and a pressure sensitive element 4. The needle 3 defines a longitudinal axis A that extends from a proximal direction P towards a distal direction D.

The needle hub 2 comprises a cylindrical wall 2.1 with a closed distal end 2.1.1 including an aperture through which the needle 3 projects to an outside of the needle hub 2. The needle hub 2 further comprises a bypass channel 2.2 that is arranged adjacent the needle 3 inside the wall of the closed distal end 2.1.1.

The bypass channel 2.2 is fluidly connected to a flow channel 3.1 of the needle 3 through which a fluid to be injected flows during a drug delivery process. The bypass channel 2.2 extends perpendicular with respect the flow channel 3.1 and is coupled with one end to the flow channel 3.1 and with another end to a recess 2.2.1 that is arranged within the wall of the distal end 2.1.1, wherein the recess 2.2.1 couples the bypass channel 3.1 with an outside of the needle hub 2.

In figure 1, the recess 2.2.1 is filled with a plug 4.1 that is positively and/or non-positively fitted within the recess 2.2.1. The plug 4.1 in the recess 2.2.1 forms the pressure sensitive element 4. If an occlusion occurs in the flow channel 3.1 as it is shown in figure 2, a pressure inside the flow channel 3.1 increases during the drug delivery process. An occlusion may occur by solidification or crystallization of the fluid inside the flow channel 3.1 when the needle 3 is left in-situ following use as previously described. The plug 4.1 is subjected to the increased pressure and as a consequence, the plug 4.1 is pushed out of the recess 2.2.1 as it is shown in figure 2 when the increased pressure exceeds a predetermined value.

For pressure compensation, a quantity of fluid inside the flow channel 3.1 flows through the bypass channel 2.2 to the outside of the needle hub 2. For example, the fluid leaks out to a patient's skin, making the patient aware that the needle 3 may be clogged or blocked.

The plug 4.1 may comprise or consist of a deformable material.

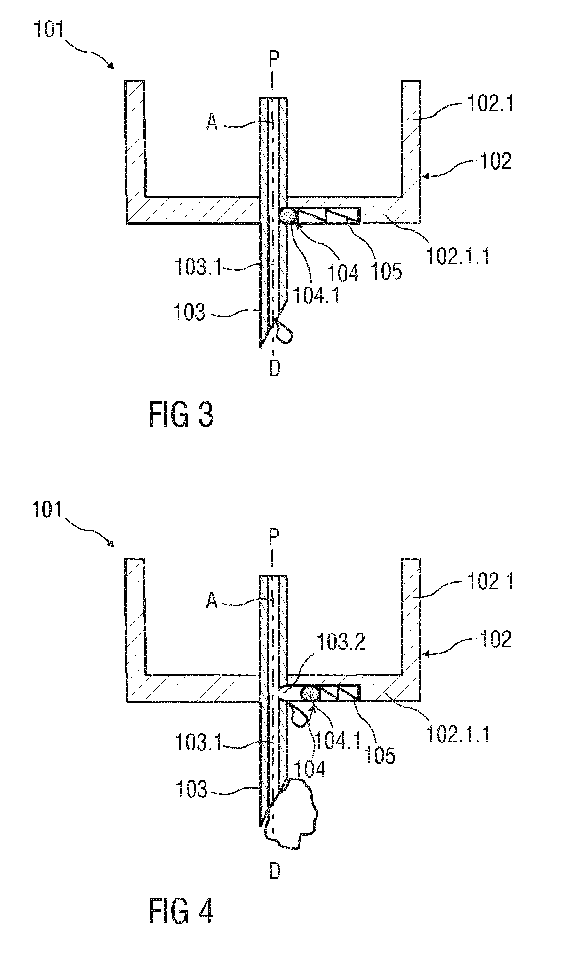

The **figures 3 and 4** show longitudinal sections of a needle assembly 101 in a further exemplary embodiment.

The needle assembly 101 comprises a needle hub 102, a hypodermic double ended hollow needle 103 held in the needle hub 102 and a pressure sensitive element 104. The needle 103 defines a longitudinal axis A that extends from a proximal direction P towards a distal direction D

The needle hub 102 comprises a cylindrical wall 102.1 with a closed distal end 102.1.1 including an aperture through which the needle 103 projects to an outside of the needle hub 102.

The needle 103 comprises a flow channel 3.1 through which a fluid to be injected flows during a drug delivery process. The needle 103 further comprises an opening 103.2 that couples the flow channel 103.1 with an outside of the needle hub 102. The opening 103.2 is arranged through a lateral surface area of the needle 103 in a section nearly on the same level as the distal end 102.1.1 along the longitudinal axis A.

In figure 3, a plug 104.1 is positively and/or non-positively fitted within the opening 103.2. The plug 104.1 is coupled to a spring 105 that is arranged within the distal end 102.1.1. The spring 105 is designed as a compressible spring that moves the plug 104.1 inside the distal end 102.1.1, in particular perpendicularly with respect to the flow channel 103.1. The space in which the spring is arranged and in which the plug 104.1 is movable may be a material weakening arranged on an outside surface of the distal end 102.1.1. It is also conceivable that the space is covered by a membrane permeable for water to protect the spring 105.

The plug 104.1 and the opening 103.2 form the pressure sensitive element 104. If an occlusion occurs in the flow channel 103.1 as it is shown in figure 4, a pressure inside the flow channel 103.1 increases during the drug delivery process. An occlusion may occur by solidification or crystallization of the fluid inside the flow channel 103.1 when the needle 103 is left in-situ following use as previously described. The plug 104.1 is subjected to the increased pressure and as a consequence, the plug 104.1 is pushed out of the opening 103.2 against the bias of the spring 105 as it is shown in figure 4 when the increased pressure exceeds a predetermined value.

For pressure compensation, a quantity of fluid inside the flow channel 103.1 flows through the opening 103.2 to the outside of the needle hub 102. For example, the fluid leaks out to a patient's skin, making the patient aware that the needle 103 may be clogged or blocked.

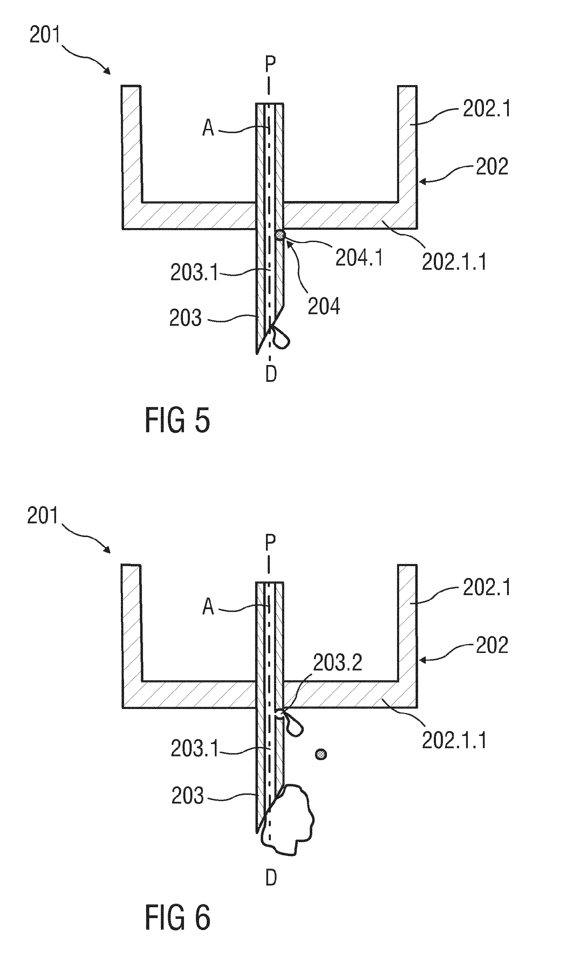

The **figures 5 and 6** show longitudinal sections of a needle assembly 201 in a further exemplary embodiment.

The needle assembly 201 comprises a needle hub 202, a hypodermic double ended hollow needle 203 held in the needle hub 202 and a pressure sensitive element 204. The needle assembly 201 defines a longitudinal axis A that extends from a proximal direction P towards a distal direction D.

The needle hub 202 comprises a cylindrical wall 202.1 with a closed distal end 202.1.1 including an aperture through which the needle 203 projects to an outside of the needle hub 202.

The needle 203 comprises a flow channel 3.1 through which a fluid to be injected flows during a drug delivery process. The needle 203 further comprises an opening 203.2 that couples the flow channel 203.1 with an outside of the needle hub 202. According to the present exemplary embodiment, the opening 203.2 is arranged through a lateral surface area of the needle 203 distally from the distal end 202.1.1.

In figure 5, a plug 204.1 is positively and/or non-positively fitted within the opening 203.2. The plug 204.1 and the opening 203.2 form the pressure sensitive element 204. If an occlusion occurs in the flow channel 203.1 as it is shown in figure 6, a pressure inside the flow channel 203.1 increases during the drug delivery process. An occlusion may occur by solidification or crystallization of the fluid inside the flow channel 203.1 when the needle 203 is left in-situ following use as previously described. The plug 204.1 is subjected to the increased pressure and as a consequence, the plug 204.1 is pushed out of the opening 203.2 as it is shown in figure 6 when the increased pressure exceeds a predetermined value.

For pressure compensation, a quantity of fluid inside the flow channel 203.1 flows through the opening 203.2 to the outside of the needle hub 202. For example, the fluid leaks out to a patient's skin, making them aware that the needle 203 may be clogged or blocked.

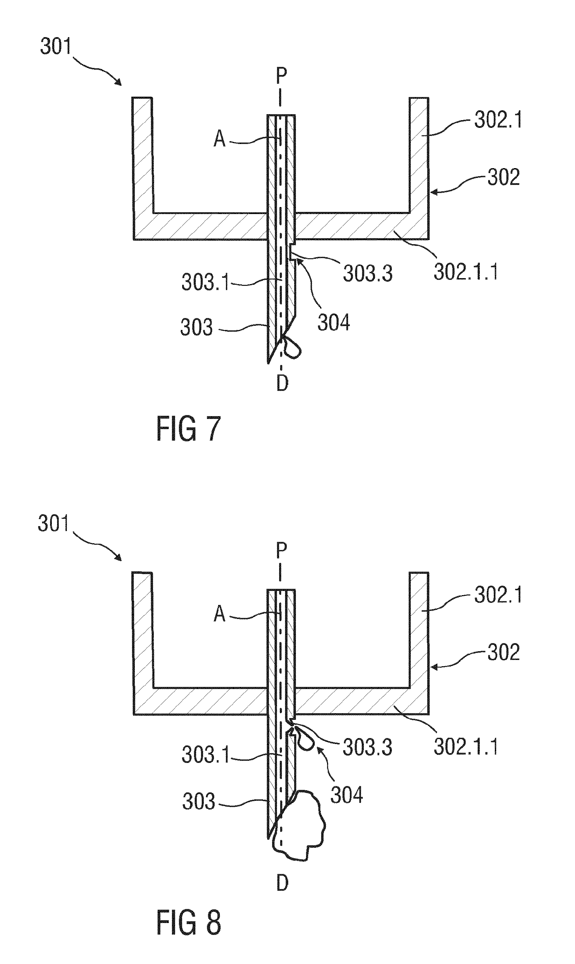

The **figures 7 and 8** show longitudinal sections of a needle assembly 301 in a further exemplary embodiment.

The needle assembly 301 comprises a needle hub 302, a hypodermic double ended hollow needle 303 held in the needle hub 302 and a pressure sensitive element 304. The needle 303 defines a longitudinal axis A that extends from a proximal direction P towards a distal direction D.

The needle hub 302 comprises a cylindrical wall 302.1 with a closed distal end 302.1.1 including an aperture through which the needle 303 projects to an outside of the needle hub 302.

The needle 303 comprises a flow channel 303.1 through which a fluid to be injected flows during a drug delivery process. The needle 303 further comprises a predetermined breaking point 303.3 that is shown in figure 7. The predetermined breaking point 303.3 is designed as a material weakening in the outer circumference of the needle 303. According to the present exemplary embodiment, the predetermined breaking point 303.3 is arranged distally from the distal end 302.1.1. Here, the predetermined breaking point 303.3 forms the pressure sensitive element 304.

If an occlusion occurs in the flow channel 303.1 as it is shown in figure 8, a pressure inside the flow channel 303.1 increases during the drug delivery process. An occlusion may occur by solidification or crystallization of the fluid inside the flow channel 303.1 when the needle 303 is left in-situ following use as previously described. If the increased pressure exceeds a predetermined value, the predetermined breaking point 303.3 breaks. The predetermined pressure value depends on a wall thickness of the predetermined breaking point 303.3.

For pressure compensation, a quantity of fluid inside the flow channel 303.1 flows through the opened predetermined breaking point 303.3 to the outside of the needle 303 and depending on the position of the predetermined breaking point 303.3 distally below the distal end 302.1.1 to the outside of the needle hub 302. For example, the fluid leaks out to a patient's skin, making them aware that the needle 303 may be clogged or blocked.

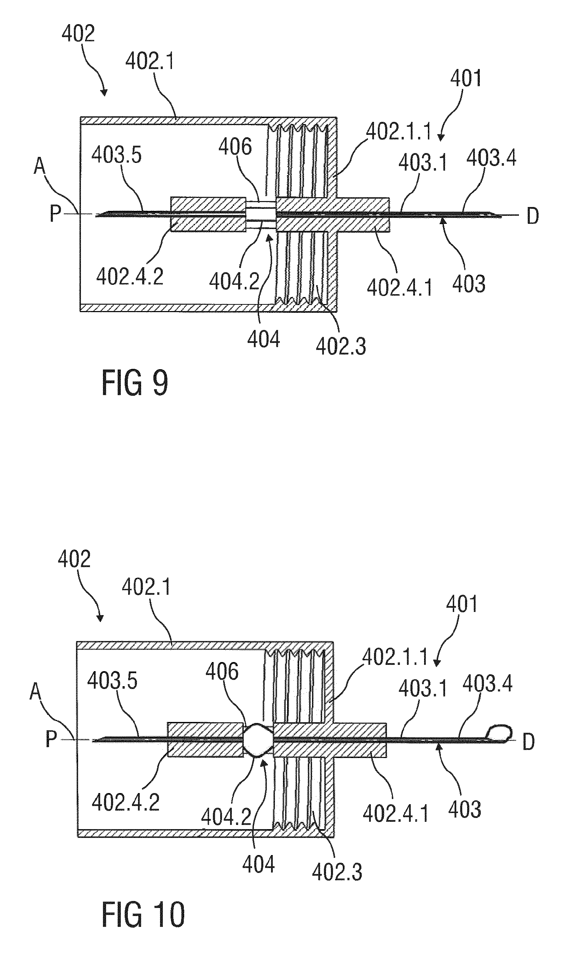

The **figures 9 and 10** show longitudinal sections of a needle assembly 401 in a further exemplary embodiment.

The needle assembly 401 comprises a needle hub 402, a hypodermic double ended hollow needle 403 held in the needle hub 402 and a pressure sensitive element 404. The needle 403 defines a longitudinal axis A that extends from a proximal direction P towards a distal direction D

The needle hub 402 comprises a cylindrical wall 402.1 with a closed distal end 402.1.1 and an internal thread 402.3.

The needle 403 comprises a flow channel 403.1 through which a fluid to be injected flows during a drug delivery process. The needle 403 is split into two segments, i. e. a distal segment 403.4 and a proximal segment 403.5, wherein both segments 403.4, 403.5 are respectively held in an elongated central boss 402.4.1, 402.4.2 of the needle hub 402. The segments 403.4, 403.5 as well as the bosses 402.4.1, 402.4.2 are axially spaced from each other by a flexible section 404.2 that is arranged between the bosses 402.4.1, 402.4.2. The flexible section 404.2 forms the pressure sensitive element 404, wherein a diameter of the flexible section 404.2 is flexible depending on a pressure in the flow channel 403.1. For example, the flexible section 404.2 is designed as a tube comprising a flexible material, e.g. rubber.

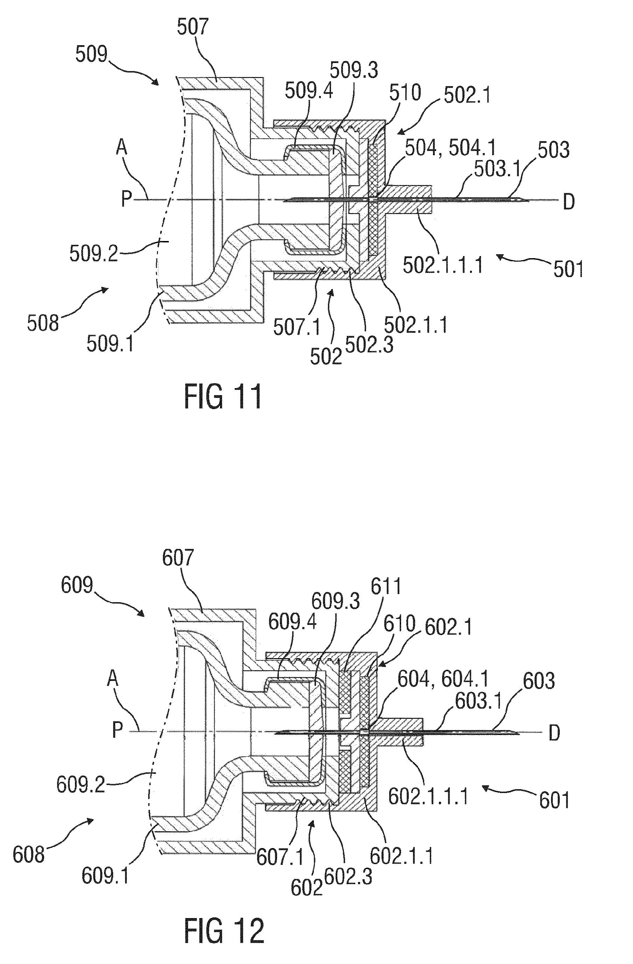

At least one sensor 406 is arranged within the needle assembly 401. The sensor 406 may be arranged adjacent the flexible section 404.2 and coupled with a lateral surface area of the flexible section 404.2. Alternatively, the sensor 406 is arranged in the area of a proximal or distal end of the needle 403. In a further alternatively embodiment, the sensor 406 is arranged in a cartridge holder 507, 607 of a drug delivery device 508, 608 (shown in figures 11 and 12). The sensor 406 is adapted to detect a variation of the diameter of the flexible section 404.2. For this purpose, the sensor 404.2 may be an optical, ultrasonic, magnetic or electrostatic sensor or comprise a combination of two or more of these detection principles.

If an occlusion occurs in the flow channel 403.1 as it is shown in figure 10, a pressure inside the flow channel 403.1 increases during the drug delivery process. An occlusion may occur by solidification or crystallization of the fluid inside the flow channel 403.1 when the needle 403 is left in-situ following use as previously described. If the pressure inside the flow channel 403.1 increases, the flexible section 404.2 begins to bulge and thus, the diameter of the flexible section 404.2 increases as well.

The bulging of the flexible section 404.2 and/or the increased diameter of the bulged flexible section 404.2 is detected by the sensor 406. If the increased diameter of the flexible section 404.2 exceeds a predetermined value, the sensor 406 may generate a signal for a visual, audible and/or tactile feedback. Therefore, at least one output unit (not shown) may be arranged within or coupled to the needle assembly 401 as an external unit. Alternatively or in addition to, the sensor 406 is coupled to an actor (not shown) that is adapted to block the drug delivery device 508, 608 in such a manner that the use of the clogged needle 403 is not possible if the sensor 406 detects a bulging of the flexible section 404.2. After replacement of the clogged needle 403, the drug delivery device 508, 608 is enabled by the actor.

**Figure 11** show a longitudinal section of a needle assembly 501 in a further exemplary embodiment.

The needle assembly 501 comprises a needle hub 502, a hypodermic double ended hollow needle 503 held in the needle hub 502 and a pressure sensitive element 504. The needle 503 defines a longitudinal axis A that extends from a proximal direction P towards a distal direction D

The needle assembly 501 is mounted to a cartridge holder 507 forming a drug delivery device 508. A distal end of the cartridge holder 507 comprises an external thread 507.1 for engaging an internal thread 502.3 of the needle hub 502 of the needle assembly 501. A medicament cartridge 509 is arranged within the cartridge holder 507. The cartridge 509 comprises a hollow body 509.1 defining a cavity 509.2 within, the cavity 509.2 being arranged for containing a fluid to be injected. A distal end of the cartridge 509 comprises a septum 509.3. A ferrule 509.4 is arranged to hold the septum 509.3 on the body 509.1.

The needle hub 502 comprises a cylindrical wall 502.1 with a distal end 502.1.1 and an internal thread 502.3. The distal end 502.1.1 comprises a stem 502.1.1.1 projecting distally from the distal end 502.1.1. The stem 502.1.1.1 comprises an aperture for receiving and guiding the needle 503, wherein a distal tip of the needle 503 extends distally from the stem 502.1.1.1.

The needle 503 comprises a flow channel 503.1 through which a fluid to be injected flows during a drug delivery process. The needle 503 further comprises a valve 504.1 as the pressure sensitive element 504. The valve 504.1 is arranged within an opening (not shown) of the needle 504. The opening of the needle 504 is arranged nearly on the same level as the distal end 502.1.1, wherein a first inner indicator 510 is arranged on an inner bottom surface of the distal end 502.1.1. Alternatively, the first inner indicator 510 is arranged within the wall of the distal end 502.1.1. The first inner indicator 510 is fluidly connected to the flow channel 503, if the valve 504.1 is opened.

The valve 504.1 opens if the needle 503 is clogged. If an occlusion of the needle 503 occurs in the flow channel 503.1, a pressure inside the flow channel 503.1 increases during the drug delivery process. An occlusion may occur by solidification or crystallization of the fluid inside the flow channel 503.1 when the needle 503 is left in-situ following use as previously described.

The valve 504.1 is subjected to the increased pressure and as a consequence, the valve 504.1 opens when the increased pressure exceeds a predetermined value.

Subsequently, the fluid inside the flow channel 503.1 gets in contact with the first inner indicator 510. The first inner indicator 510 may comprise a chemical indicator, such as an acid and/or base sensitive indicator. The contact of the fluid with the first inner indicator 510 results in a colour change of the first inner indicator 510. The colour change may indicate to a user that not all of the fluid has been injected.

**Figure 12** shows a longitudinal section of a needle assembly 601 in a further exemplary embodiment.

The needle assembly 601 comprises a needle hub 602, a hypodermic double ended hollow needle 603 held in the needle hub 602 and a pressure sensitive element 604. The needle 603 defines a longitudinal axis A that extends from a proximal direction P towards a distal direction D.

The needle assembly 601 is mounted to a cartridge holder 607 forming a drug delivery device 608. A distal end of the cartridge holder 607 comprises an external thread 607.1 for engaging an internal thread 602.3 of the needle hub 602 of the needle assembly 601. A medicament cartridge 609 is arranged within the cartridge holder 607. The cartridge 609 comprises a hollow body 609.1 defining a cavity 609.2 within, the cavity 609.2 being arranged for containing a fluid to be injected. A distal end of the cartridge 609 comprises a septum 609.3. A ferrule 609.4 is arranged to hold the septum 609.3 on the body 609.1.

The needle hub 602 comprises a cylindrical wall 602.1 with a distal end 602.1.1 and an internal thread 602.3. The distal end 602.1.1 comprises a stem 602.1.1.1 projecting distally from the distal end 602.1.1. The stem 602.1.1.1 comprises an aperture for receiving and guiding the needle 603, wherein a distal tip of the needle 603 extends distally from the stem 602.1.1.1.

The needle 603 comprises a flow channel 603.1 through which a fluid to be injected flows during a drug delivery process. The needle 603 further comprises a valve 604.1 as the pressure sensitive element 604. The valve 604.1 is arranged within an opening (not shown) of the needle 604. The opening of the needle 604 is arranged nearly on the same level as the distal end 602.1.1, wherein a first inner indicator 610 is arranged on an inner bottom surface of the distal end 602.1.1. Alternatively, the first inner indicator 610 is arranged within the wall of the distal end 602.1.1. The first inner indicator 610 is fluidly connected to the flow channel 603, if the valve 604.1 is opened.

The valve 604.1 opens if the needle 603 is clogged. If an occlusion of the needle 603 occurs in the flow channel 603.1, a pressure inside the flow channel 603.1 increases during the drug delivery process. An occlusion may occur by solidification or crystallization of the fluid inside the flow channel 603.1 when the needle 603 is left in-situ following use as previously described. The valve 604.1 is subjected to the increased pressure and as a consequence, the valve 604.1 opens when the increased pressure exceeds a predetermined value.

Subsequently, the fluid inside the flow channel 603.1 gets in contact with the first inner indicator 610. The first inner indicator 610 may comprise a chemical indicator, such as an acid and/or base sensitive indicator. The contact of the fluid with the first inner indicator 610 results in a colour change of the first inner indicator 610. The colour change may indicate to a user that not all of the fluid has been injected.

The drug delivery device 608 comprises a second inner indicator 611 that is arranged proximally behind the first indicator 610 and at a proximal distance therefrom. The second inner indicator 611 is fluidly sealed from the first inner indicator 610 and thus not in fluid connection with the flow channel 603.1 if the valve 604.1 is open. The second inner indicator 611 is adapted to indicate a leakage of the fluid from the cartridge 609. Hence, the present embodiment is suited to differentiate between a clogged or blocked needle 603 and a leaking cartridge 609.

A further not shown exemplary embodiment is based on the embodiment according to figure 12. Here, the drug delivery device 608 includes an additional outer indicator arranged on an outside of the drug delivery device 608. The outer indicator is adapted to indicate a leakage of the fluid out of the drug delivery device 608, in particular on a skin of a user.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyldes(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Γ-glutamyl)-des(B30) human insulin; B29-N-(N-lithocholyl-Γ-glutamyl)-des(B30) human insulin; B29-N-(ω-carboxyheptadecanoyl)-des(B30) human insulin and B29-N-(ω-carboxyheptadecanoyl) human insulin.

Exendin-4 for example element Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   des Pro36 Exendin-4(1-39)-Lys6-NH2 (AVE0010),
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2,
   H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or A, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

The term "drug delivery device" shall be understood to encompass any type of device, system or apparatus which may serve to dispense a drug or a formulation containing a drug immediately to a human or veterinarian body. Immediately dispense thereby shall be understood as an absence of any necessary intermediate manipulation of the drug or drug formulation by a user between discharge of the drug or drug formulation from the drug delivery device and administration to the human or veterinarian body. Without limitation, typical examples of an immediate dispensing device may be found in injection devices, inhalers and stomach tube feeding systems.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: needle assembly
- 2: needle hub
- 2.1: cylindrical wall
- 2.1.1: distal end
- 2.2: bypass channel
- 2.2.1: recess
- 3: needle
- 3.1: flow channel
- 4: pressure sensitive element
- 4.1: plug

- 101: needle assembly
- 102: needle hub
- 102.1: cylindrical wall
- 102.1.1: distal end
- 103: needle
- 103.1: flow channel
- 103.2: opening
- 104: pressure sensitive element
- 104.1: plug
- 105: spring

- 201: needle assembly
- 202: needle hub
- 202.1: cylindrical wall
- 202.1.1: distal end
- 203: needle
- 203.1: flow channel
- 203.2: opening
- 204: pressure sensitive element
- 204.1: plug

- 301: needle assembly
- 302: needle hub
- 302.1: cylindrical wall
- 302.1.1: distal end
- 303: needle
- 303.1: flow channel
- 303.3: predetermined breaking point
- 304: pressure sensitive element

- 401: needle assembly
- 402: needle hub
- 402.1: cylindrical wall
- 402.1.1: distal end
- 402.3: internal thread
- 402.4.1, 402.4.2: boss
- 403: needle
- 403.1: flow channel
- 403.4: distal segment
- 403.5: proximal segment
- 404: pressure sensitive element
- 404.2: flexible section
- 406: sensor

- 501: needle assembly
- 502: needle hub
- 502.1: cylindrical wall
- 502.1.1: distal end
- 502.3: internal thread
- 503: needle
- 503.1: flow channel
- 504: pressure sensitive element
- 504.1: valve
- 507: cartridge holder
- 508: drug delivery device
- 509: cartridge
- 509.1: body
- 509.2: cavity
- 509.3: septum
- 509.4: ferrule
- 510: inner indicator

- 601: needle assembly
- 602: needle hub
- 602.1: cylindrical wall
- 602.1.1: distal end
- 602.3: internal thread
- 603: needle
- 603.1: flow channel
- 604: pressure sensitive element
- 604.1: valve
- 607: cartridge holder
- 608: drug delivery device
- 609: cartridge
- 609.1: body
- 609.2: cavity
- 609.3: septum
- 609.4: ferrule
- 610: inner indicator
- 611: further inner indicator

- A: longitudinal axis
- D: distal direction
- P: proximal direction

## Claims

1. Needle assembly (1, 101, 201, 301, 401, 501, 601), comprising a hollow needle (3, 103, 203, 303, 403, 503, 603) defining a
flow channel (3.1, 103.1, 203.1, 303.1, 403.1, 503.1, 603.1) and a needle hub (2, 102, 202, 302, 402, 502, 602),
**characterized by** at least one pressure sensitive
element (4, 104, 204, 304, 404, 504, 604) that is adapted to detect an occlusion in the flow channel (3.1, 103.1, 203.1, 303.1, 403.1, 503.1, 603.1), wherein the pressure sensitive element (4, 104, 204, 304, 404, 504, 604) is modifiable if a pressure in the flow channel (3.1, 103.1, 203.1, 303.1, 403.1, 503.1, 603.1) exceeds a predetermined value.

2. Needle assembly (1, 101, 201, 501, 601) according to claim 1,
**characterized in that** the pressure sensitive element (4, 104, 204, 504, 604) comprises a valve that is configured for opening if the pressure in the flow channel (3.1, 103.1, 203.1, 503.1, 603.1) exceeds the predetermined value.

3. Needle assembly (1) according to claim 2,
**characterized in that** the valve comprises a plug (4.1) that is form-lockingly arranged within a recess (2.2.1) that is arranged in a bypass channel (2.2) of the needle hub (2), wherein the bypass channel (2.2) is fluidly connected to the flow channel (3.1), and wherein the plug (4.1) takes off the recess (2.2.1) if the pressure in the flow channel (3.1) exceeds the predetermined value.

4. Needle assembly (1) according to claim 3,
**characterized in that** the recess (2.2.1) couples the bypass channel (2.2) with an outside of the needle hub (2).

5. Needle assembly (101) according to claim 2,
**characterized in that** the valve comprises a plug (104.1) that is coupled to a spring (105), wherein the plug (104.1) is positively and/or non-positively fitted within an opening (103.2) that is arranged in a lateral surface of the needle (103), and wherein the plug (104.1) is pushed out of the opening (103.2) if the pressure in the flow channel (103.1) exceeds the predetermined value.

6. Needle assembly (101) according to claim 5,
**characterized in that** the spring (105) is movable perpendicularly with respect to the flow channel (103.1), wherein the spring (105) is tensioned when the plug (104.1) is pushed out of the opening (103.2).

7. Needle assembly (501) according to claim 2,
**characterized in that** the valve is coupled to an inner indicator (510) that is arranged within the needle hub (502) and fluidly connected to the flow channel (503.1) when the valve is opened.

8. Needle assembly (501) according to claim 7,
**characterized in that** the inner indicator (510) generates a visual and/or audible and/or tactile feedback when the valve is opened.

9. Needle assembly (501) according to claim 8,
**characterized in that** the inner indicator (510) comprises a chemical indicator.

10. Needle assembly (601) according to claim 2,
**characterized in that** the valve is coupled to an inner indicator (610) and wherein a further inner indicator (611) is arranged, wherein
- the inner indicator (610) generates a visual and/or audible and/or tactile feedback when the valve is opened and
- the further inner indicator (611) generates a visual and/or audible and/or tactile feedback when a fluid leaks out of a cartridge (609) of a drug delivery device (608).

11. Needle assembly (301) according to claim 1,
**characterized in that** the pressure sensitive element (304) comprises a predetermined breaking point (303.3) that is arranged on a lateral surface of the needle (303), wherein the predetermined breaking point (303.3) breaks if the pressure within the flow channel (303.1) exceeds the predetermined value.

12. Needle assembly (401) according to claim 1,
**characterized in that** pressure sensitive element (404) comprises a flexible section (404.2) that forms a section of the flow channel (403.1) with a variable diameter, wherein the diameter of the flexible section (404.2) increases if the pressure in the flow channel exceeds the predetermined value.

13. Needle assembly (401) according to claim 12,
**characterized in that** the diameter of the flexible section (404.2) is detectable by a sensor (406).

14. Needle assembly (401) according to claim 12 or 13,
**characterized in that** a visual, audible and/or tactile feedback is issued if the sensor (406) detects a diameter of the flexible section (404.2) exceeding a predetermined value.
